# EUROPEAN PATENT SPECIFICATION

(11) **EP 0 495 939 B1**
(45) Date of publication and mention of the grant of the patent: **27.11.1996**
(21) Application number: 91912303.4
(22) Date of filing: 21.06.1991
(51) Int. Cl.: G01N 33/68, C07K 1/10, C08J 5/00

(54) **SEQUENTIAL C-TERMINAL DEGRADATION OF PEPTIDES AND PROTEINS**
SEQUENZIELLE C-TERMIN DEGRADIERUNG VON PEPTIDEN UND PROTEINEN
DEGRADATION SEQUENTIELLE DE LA TERMINAISON C DE PEPTIDES ET DE PROTEINES

(30) Priority: 13.08.1990 US 576943
(43) Date of publication of application: 29.07.1992
(60) Divisional application: 96200251.5
(73) Proprietor: CITY OF HOPE, Duarte California 91010-0269 (US)
(72) Inventor: BAILEY, Jerome, M., Duarte, CA 91010 (US)
(74) Representative: Dowden, Marina
(86) International application number: PCT/US91/04434
(87) International publication number: WO 92/03737

(56) References cited:
- WO-A-91/18008
- GB-A- 2 082 599
- US-A- 4 582 875
- US-A- 4 837 165
- US-A- 4 935 494
- POLYMER BULLETIN, vol. 3, 1980, Heidelberg (DE); C. G. BEDDOWS et al., pp. 645- 653/
- ANGEWANDTE CHEMIE, INTERNATIONAL EDITION, vol. 18, no. 10, October 1979, Weinheim (DE); T. MUKAIYAMA, pp. 707-721/

## Description

This invention relates to the sequential degradation of peptides and proteins from the carboxy-terminus. More particularly, the invention relates to a method for the cleavage of the C-terminal thiohydantoin amino acid from the derivatized peptide which results from the use of a silylisothiocyanate as a coupling reagent in carboxy-terminal sequencing procedures.

### A. Background

The development of methods for the sequential degradation of proteins and peptides from the carboxy-terminus has been the objective of several studies. See Ward, C.W., Practical Protein Chemistry - A Handbook (Darbre, A., ed.) (1986) and Rangarajan, M., Protein/Peptide Sequence Analysis: Current (1988). Such a method would complement existing N-terminal degradations based on the Edman chemistry. Edman, P., Acta.Chem.Scand. 4:283-293 (1950). The most widely studied method and probably the most attractive because of its similarity to the Edman degradation has been the conversion of amino acids into thiohydantoins. This reaction, originally observed by Johnson and Nicolet, J.Am.Chem.Soc. 33:1973-1978 (1911), was first applied to the sequential degradation of proteins from the carboxy-terminus by Schlack and Kumpf, Z.Physiol.Chem. 154:125-170 (1926). These authors reacted ammonium thiocyanate, dissolved in acetic acid and acetic anhydride, with N-benzoylated peptides to form carboxyl-terminal 1-acyl-2-thiohydantoins. Exposure to strong base was used to liberate the amino acid thiohydantoin and generate a new carboxyl-terminal amino acid. The main disadvantages of this procedure have been the severity of the conditions required for complete derivatization of the C-terminal amino acid and for the subsequent cleavage of the peptidylthiohydantoin derivative into a new shortened peptide and an amino acid thiohydantoin derivative.

Since this work was published, numerous groups have tried to reduce the severity of the conditions required, particularly in the cleavage of the peptidylthiohydantoin, in order to apply this chemistry to the sequential degradation of proteins from the carboxyl terminal end. Lesser concentrations of sodium hydroxide than originally used by Schlack and Kumpf and of barium hydroxide were found to effectively cleave peptidylthiohydantoins. See Waley, S.G., et al., J.Chem.Soc. 1951:2394-2397 (1951); Kjaer, A., et al., Acta Chem.Scand. 6:448-450 (1952); Turner, R.A., et al., Biochim.Biophys.Acta. 13:553-559 (1954). Other groups used acidic conditions based on the original procedure used by Johnson and Nicolet for the de-acetylation of amino acid thiohydantoins. See Tibbs, J., Nature 168:910 (1951); Baptist, V.H., et al., J.Am.Chem.Soc. 75:1727-1729 (1953). These authors added concentrated hydrochloric acid to the coupling solution to cause cleavage of the peptidylthiohydantoin bond. Unlike hydroxide which was shown to cause breakdown of the thiohydantoin amino acids, hydrochloric acid was shown not to destroy the amino acid thiohydantoins. See Scoffone, E., et al., Ric.Sci. 26:865-871 (1956); Fox, S.W., et al., J.Am.Chem. Soc. 77:3119-3122 (1955); Stark, G.R., Biochem. 7:1796-1807 (1968). Cromwell, L.D., et al., Biochem. 8:4735-4740 (1969) showed that the concentrated hydrochloric acid could be used to cleave the thiohydantoin amino acid at room temperature. The major drawback with this procedure was that when applied to proteins, no more than two or three cycles could be performed.

Yamashita, S., Biochem.Biophys.Acta. 229:301-309 (1971) found that cleavage of peptidylthiohydantoins could be done in a repetitive manner with a protonated cation exchange resin. Application of this procedure to 100 µmol quantities of papain and ribonuclease was reported to give 14 and 10 cycles, respectively, although no details were given. See Yamashita, S., et al., Proc.Hoshi.Pharm. 13:136-138 (1971). Stark reported that certain organic bases, such as morpholine or piperidine, could be substituted for sodium hydroxide, and along the same lines, Kubo, H., et al., Chem.Pharm.Bull. 19:210-211 (1971) reported that aqueous triethylamine (0.5M) could be used to effectively cleave peptidylthiohydantoins. Stark appeared to have solved the cleavage problem by introducing acetohydroxamic acid in aqueous pyridine at pH 8.2 as a cleavage reagent. This reagent was shown to rapidly and specifically cleave peptidylthiohydantoins at room temperature and at mild pH.

Conditions for the formulation of the peptidylthiohydantoins were improved by Stark and Dwulet, F.E., et al., Int.J.Peptide and Protein Res. 13:122-129 (1979), who reported on the use of thiocyanic acid rather than thiocyanate salts, and more recently by the introduction of trimethylsilylisothiocyanate (TMS-ITC) as a coupling reagent. See Hawke, D.H., et al., Anal.Biochem. 166:298-307 (1987). The use of this reagent for C-terminal sequencing has been patented. See Hawke patent United States 4,837,165. This reagent significantly improved the yields of peptidylthiohydantoin formation and reduced the number of complicating side products. Cleavage of peptidylthiohydantoins by 12 N HCl (Hawke, 1987) and by acetohydroxamate (Miller, C.G., et al., Techniques in Protein Chemistry (Hugli, T.E., ed.) pp. 67-68, Academic Press (1989)) failed to yield more than a few cycles of degradation.

### B. The Cleavage Problem

Although the cleavage reaction has been extensively studied since the thiocyanate chemistry for C-terminal degradation was first proposed by Schlack and Kumpf in 1926, a chemical method has not yet been proposed that is capable of an extended degradation. Cleavage in 1N sodium hydroxide as first proposed by Schlack and Kumpf (1926) is well known to hydrolyze proteins and peptides at other sites in addition to cleavage of the C-terminal peptidylthiohydantoin. The released thiohydantoin amino acid derivatives are also known to be unstable in hydroxide solutions. Scoffone, supra. Cleavage by hydroxide is known to convert the side chain amide groups of asparagine and glutamine residues to a carboxylic group making these residues indistinguishable from aspartate and glutamate, respectively.

When cleavage of peptidylthiohydantoins by 12N HCl was applied to proteins and peptides no more than 2 or 3 cycles could be performed. See, Cromwell, supra and Hawke, supra. This was probably due to differences in the rate of hydrolysis of peptidylthiohydantoins containing different amino acid side chains as well as to hydrolysis of other internal amide bonds. Likewise, during the synthesis of the standard amino acid thiohydantoin derivatives corresponding to the naturally occurring amino acids, it was observed that the rate of deacetylation of the N-acetylthiohydantoin amino acids by 12 HCl depended on the nature of the amino acid side chain. Bailey, J.M., et al. Biochem. 29:3145-3156 (1990).

Attempts by Dwulet, supra, to reproduce the resin based cleavage method of Yamashita, supra, was reported to be unsuccessful. Cleavage of peptidylthiohydantoins with aqueous methanesulfonic acid was also attempted by Dwulet and by Bailey, et al., both without success. Methanesulfonic acid was chosen since it is equivalent to the acidic group on the resin employed by Yamashita (1971) and Yamashita, et al. (1971).

Cleavage of the peptidylthiohydantoin derivatives with acetohydroxamate as originally reported by Stark, supra, was found to result in the formation of stable hydroxamate esters at the C-terminus of the shortened peptide (Bailey, et al., supra). Depending on the conditions employed, between 68% and 93% of the peptide was derivatized at the C-terminus and thus prevented from further sequencing. Although Stark, supra, predicted such hydroxamate esters to form as an intermediate during cleavage, it was assumed that they would break down under the conditions used for cleavage or continued sequencing. The peptidyl hydroxamate esters formed from cleavage with acetohydroxamate, like the hydroxamate esters studied by Stieglitz, J., et al., J.Am.Chem.Soc. 36:272-301 (1914) and Scott, A.W., et al., J.Am.Chem.Soc. 49:2545-2549 (1927), are stable under the acidic conditions used for thiohydantoin formation and can only be hydrolyzed to a free peptidyl carboxylic group, capable of continued sequencing, under strongly basic conditions. This probably explains the low repetitive yields of Stark, supra; Meuth, J.L., et al., Biochem. 21:3750-3757 (1982) and Miller, supra, when aqueous acetohydroxamate was employed as a cleavage reagent.

Cleavage of peptidylthiohydantoins by aqueous triethylamine was originally reported by Kubo, H., et al., Chem.Pharm.Bull. 19:210-211 (1971), Dwulet, et al., supra, and Meuth, et al., supra. The latter group commented on the usefulness of triethylamine as a cleavage reagent for automated sequencing because of its volatility, but declined to pursue this method apparently in favor of cleavage by acetohydroxamate. Cleavage of peptidylthiohydantoins, in the solution phase, by a 2% aqueous solution of triethylamine was found to be rapid (half-times of 1 min. and 5 min. at 37°C and 22°C, respectively) and quantitative, yielding only shortened peptide capable of continued sequencing and the amino acid thiohydantoin derivative. Bailey, et al., supra.

The automation of C-terminal sequencing requires prolonged (1 to 10 days) storage of reagents in glass bottles at room temperature within the instrument. The reagents used for sequencing must be stable to these conditions. Storage of triethylamine in water rapidly results in the breakdown of the triethylamine. These breakdown products include primary and secondary amines which can subsequently block the shortened peptide from further sequencing. Free radical compounds are also formed during the breakdown of triethylamine. These free radical compounds are often UV absorbing and can interfere with the subsequent HPLC detection of the released thiohydantoin amino acid. Applicant's experience with using aqueous triethylamine (5% triethylamine in water) for automated C-terminal sequencing has consistently resulted in repetitive yields no higher than 60%, thereby permitting no more than three cycles of C-terminal degradation to be performed on peptides covalently coupled to PVDF or polyethylene membrane supports.

WO 91/18008 is a document which is relevant only under Article 54(3) and (4) EPC since it was not published until after the priority date and international filing date of the present case. This patent discloses a method for sequencing a peptide by carboxyl terminal degradation which comprises coupling the carboxyl terminus of a peptide with a silyl isothiocyanate coupling reagent to form a peptidylthiohydantoin derivative which is cleaved with sodium trimethylsilanolate.

### C. Peptide Sample Supports

In the preferred practice of the C-terminal sequencing chemistry of this invention, the peptide sample is covalently attached to a solid support. Applicant and others (Inglis et al., Met. Protein sequence Analysis (Jornavall/Hoog/Gustavsson, Eds.) pp. 23-24, Birkhauser-Verlag, Basel (1991); Wittman-Liebold, et al., Met. Protein Sequence Analysis (Jornavall/Hoog/Gustavsson, Eds.) pp. 9-21, Birkhauser-Verlag, Basel (1991); Hawke and Boyd, Met. Protein Sequence Analysis (Jornavall/Hoog/Gustavsson, Eds.) pp. 35-45, Birkhauser-Verlag, Basel (1991)), have recognized that C-terminal chemistry is preferably applied to samples covalently attached, at the N-terminal, to a solid phase. Covalent immobilization of the sample on a solid support allows the use of reagents and solvents optimal for sequencing without causing sample washout, the capability to efficiently wash the sample to remove reaction by-products which might otherwise interfere with identification of the released thiohydantoin amino acids, and prevents mechanical losses associated with most solution phase methods. In general, automated solid phase chemistry is expected to be more efficient and less labor intensive compared to manual solution phase methods.

Since the introduction of the solid-phase approach to N-terminal protein sequencing by Laursen, R.A. J. Amer. Chem. Soc., 88:5344-5346 (1966), several different types of functionalized supports have been described for the covalent immobilization of polypeptide samples. These include polystyrene resins, polyacrylamide resins, and glass beads substituted with aminoalkyl or aminophenyl groups (Laursen and Machleidt, Methods Biochem. Anal. 26:201-284 (1980); Machleidt, Modern Methods in Protein chemistry (Tschesche, H., Ed.) pp. 262-302, de Gruyter, Berlin/New York (1983)). Typically these amino functionalized supports are activated for protein coupling with bifunctional reagents such as phenylene diisothiocyanate (DITC). The DITC group is capable of forming a stable thiourea linkage to the support and the peptide N-terminal amino group or epsilon amino group of side chain lysines. Recently glass beads derivatized with isothiocyanato, aminophenyl and aminoethylaminopropyl groups (Song-Ping Liang and Laursen, Anal. Biochem. 188:366-373 (1990)), glass fibre sheets functionalized with aminophenyl groups (Aebersold et al., Anal. Biochem. 187:56-65 (1990)), and PVDF (polyvinylidene difluoride) membranes derivatized with aryl amines and DITC (Pappin et al., Current Research in Protein Chemistry (Villafranca, J.J., Ed.) pp. 191-202, Academic Press, Inc. (1990)) have been used for the covalent immobilization of polypeptides for N-terminal sequencing. The polypeptides are either immobilized by coupling between the epsilon amino groups of the lysine and the isothiocyanate groups on the solid support using the established DITC chemistry or by the coupling of the activated C-terminal carboxyl groups of the polypeptides and the amino groups on the matrix.

Many of the initial studies involving the application of the thiocyanate chemistry for C-terminal sequencing to the solid phase have involved the use of glass beads for the ccvalent immobilization of peptide samples (Williams and Kassall, FEBS Lett. 54:353-357 (1975); Rangarajan and Darbre, Biochem. J. 157:307-316 (1976); Meuth et al., Biochem. 21:3750-3757 (1982); Hawke et al., Anal. Biochem. 166:298-307 (1987); Inglis, et al. Methods in Protein Sequence Analysis (Wittmann-Liebold, B., Ed.) pp. 137-144, Springer-Verlag (1989). More recent work has involved the use of carboxylic acid modified PVDF (Bailey and Shively, Techniques in Protein Chemistry: II (Villafranca, J.J., Ed.) pp. 115-129, Academic Press, Inc. (1991)), DITC-activated amino PVDF (Miller et al., Techniques in Protein Chemistry (Hugli, T.E., Ed.) pp. 67-78, Academic Press, Inc. (1989), Inglis et al., Met. Protein Sequence Analysis (Jornval/Hoog/Gustavsson, Eds.) pp. 23-24, Birkhauser-Verlag, Basel (1991)), and a disuccinimidoyl carbonate polyamide resin (Hawke and Boyd, Met. Protein Sequence Analysis (Jornvall/Hoog/Gustavsson, Eds.) pp. 35-45, Birkhauser-Verlag, Basel (1991).

The use of glass and PVDF supports for C-terminal sequencing is attended by disadvantages. Siloxane bonds are formed on derivatization of the glass supports. These bonds are base labile resulting in loss of the covalently coupled peptide sample. The carboxyl modified PVDF and the DITC-activated amino PVDF are both physically and chemically altered adversely due to dehydro- fluorination (Dias and McCarthy, Macromolecules 17:2529-2531 (1984)) caused by the basic cleavage reagents used during the course of C-terminal sequencing. These membranes may turn successively darker and more brittle on each C-terminal sequencing cycle.

Polymer Bulletin, 3, (1980) pages 645 to 653 describes supports for use in enzyme immobilization. The reference describes the grafting of poly(acrylic acid) directly onto polyethylene and the use of the resultant copolymer to couple with bovine serum albumin and phenyl propylamine. In particular, this reference describes the use of N-ethoxycarbonyl-2-ethoxy,-1,2-dihydroquinoline as the coupling agent, resulting in various levels of success.

### SUMMARY OF THE INVENTION

This invention provides novel C-terminal peptide cleavage reagents, including (i) alkali metal salts of lower trialkylsilanols and (ii) trialkylamine N-oxides. These novel cleavage reagents have particular utility in sequencing procedures in which a silyl isothiocyanate is used as the coupling reagent. Sodium trimethyl silanolate and trimethyl amine N-oxide are preferred. In the preferred practice of the invention the N-terminal of the peptide sample is covalently bound to an activated PE-COOH solid phase.

Hence, the present invention relates to a method for sequencing a peptide by carboxyl terminal degradation comprising coupling the carboxyl terminus of a peptide with a silylisothiocyanate coupling reagent to form a peptidyl thiohydantoin derivative and cleaving the peptidyl thiohydantoin derivative with a reagent having the formula (R¹)₃N⁺O⁻ in which R¹ is an alkyl group having from 2 to 4 carbon atoms; to provide a thiohydantoin derivative of the amino acid previously at the carboxyl terminus of the peptide and a peptidyl residue lacking such an amino acid. According to this embodiment of the invention, the cleaving reagent is preferably trimethylamine N-oxide and in addition, the peptide may optionally be coupled to a polyvinyldifluoride or polyethylene membrane.

The present invention also provides a method for sequencing a peptide by carboxyl terminal degradation comprising coupling the carboxyl terminus of a peptide with a silylisothiocyanate coupling reagent to form a peptidyl thiohydantoin derivative with a reagent having the formula R₃SiO⁻X⁺, in which R is a straight or branched chain hydrocarbon radical having from 1 to 10 carbon atoms and X is an alkali metal ion; or the formula (R¹)₃N⁺O⁻ as defined in claim 1; to provide a thiohydantoin derivative of the amino acid previously at the carboxyl terminus of the peptide and a peptidyl residue lacking such an amino acid,
wherein the peptide is coupled to a polyvinyldifluoride or a polyethylene membrane which is carboxylic acid modified and, optionally, activated. According to this embodiment of the invention, the cleaving reagent is preferably sodium trimethylsilanolate or trimethylamine N-oxide.

### DESCRIPTION OF THE FIGURES

Figure 1 is the formula of sodium trimethylsilanolate.

Figure 2 is the formula of trimethylamine N-oxide.

Figure 3 is the postulated mechanism of the cleavage reaction with sodium trimethylsilanolate and the use of aqueous acid, e.g., trifluoracetic acid (TFA), following the cleavage reaction.

Figure 3A shows an analogous mechanism in which sodium trimethyl silanolate is used following the cleavage reaction.

Figure 4 shows the sequencing of leucine-enkephalin (YGGFL) covalently coupled to PVDF.

Figure 5 shows the sequencing from the C-terminal of KVILF covalently attached to a PVDF membrane.

Figure 6 shows the sequencing from the C-terminal of the peptide (YGGFL) covalently coupled to an activated PE-COOH membrane.

Figure 7 shows the sequencing from the C-terminal of the peptide (RGYALG) covalently coupled to an activated PE-COOH membrane.

Figure 8 shows the sequencing from the C-terminal of the peptide (YGGFMRGL) covalently coupled to an activated PE-COOH membrane.

Figure 9 shows the sequencing from the C-terminal of the peptide (YGGFL) covalently coupled to a linker 11-amino undecaonic acid.

Figure 10 is a schematic of the instrument used for C-terminal sequencing.

### DETAILED DESCRIPTION OF THE INVENTION

The mechanism of acid and base hydrolysis of acylthiohydantoins was studied in detail by Congdon and Edward, Can.J.Chem. 50:3767-3788 (1972) and a number of cleavage reagents were tested by Stark, supra. Stark found that oxygen containing nucleophiles were the best choice of reagents to effect this reaction. Although acetohydroxamate is an excellent cleavage reagent for the first amino acid, it forms a stable peptidyl hydroxamate ester, which is difficult to remove, and which effectively blocks the shortened peptide from further sequencing. This reagent also results in a high UV absorbing background during subsequent HPLC identification of the released thiohydantoin amino acids. It seems that in general any carbon based reagent that is a good nucleophile and thus a good cleavage reagent will also be a poor leaving group, thereby blocking much of the shortened peptide from further sequencing.

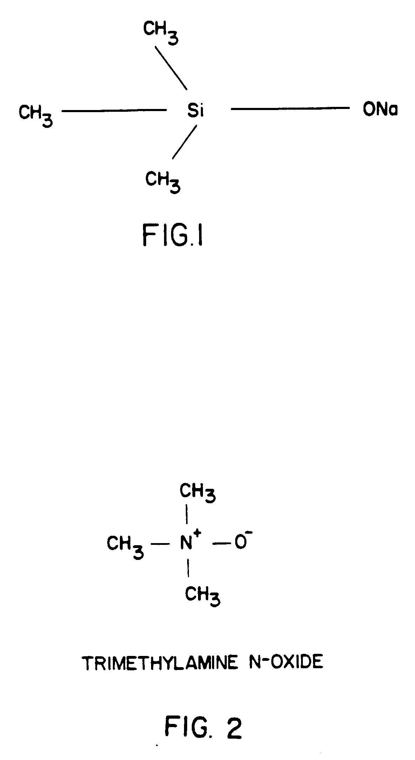

Ideally, a cleavage reagent should possess the following characteristics: (1) it should be able to cleave peptidylthiohydantoins in a volatile, water miscible organic solvent, thus eliminating the problems of incompatibility of PVDF membranes with water; (2) the reaction should be rapid and specific; (3) the shortened peptide should be capable of continued degradation; (4) the released thiohydantoin amino acid should not be destroyed by this reagent; and (5) this reagent should not absorb light in the same range as is used for detection of the released thiohydantoin amino acid derivatives. Sodium trimethylsilanolate (Figure 1), commercially available from Petrarch (Huls), in, e.g., alcoholic solvents, and trimethyl amine N-oxide (Figure 2) commercially available from Aldrich Chemical Co. in alcoholic and a wider range of solvents seem to possess all of these characteristics. Cleavage of peptidylthiohydantoins in the solution phase with a 0.05M solution of sodium trimethylsilanolate in 100% methanol or trimethylsilylethanol is complete in less than 5 minutes. A 0.05M solution of sodium trimethylsilanolate in methanol or trimethylsilylethanol effects cleavage of peptidylthiohydantoins both in the solution phase and in the solid phase in less than 5 minutes.

More specifically, this invention contemplates the use of the cleavage reagents of this invention in a concentration of from about 0.025 molar to about 0.25 molar, preferably from about 0.1 to about 0.2 molar, in methanol, trimethylsilylethanol or similar alcohols or, in the case of trimethylamine N-oxide, a wider range of solvents.

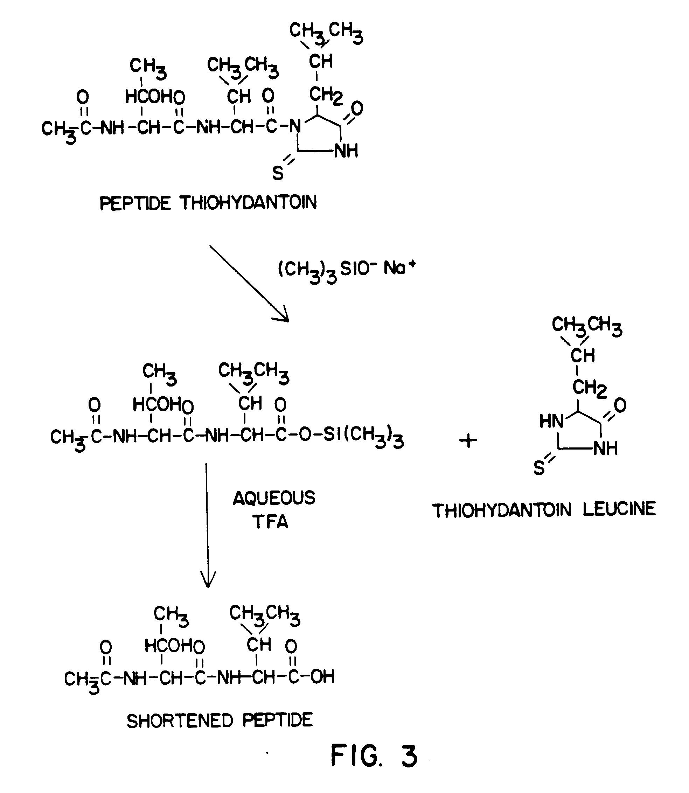

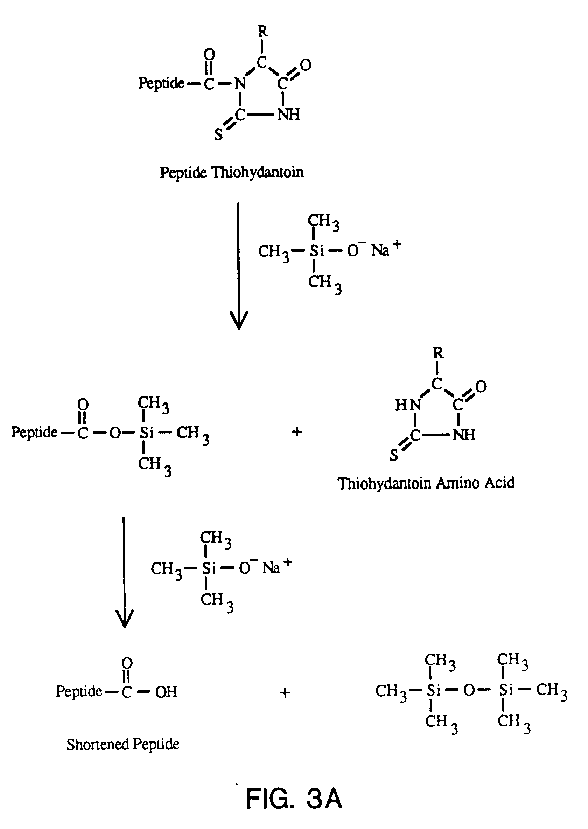

The most probable mechanism of this cleavage involves formation of an unstable C-terminal trimethylsilyl ester which, in the presence of water or alcohol, rapidly reforms the desired C-terminal carboxylic group. See Figure 3 which illustrates the use of aqueous trifluoroacetic acid (TFA) following the cleavage reaction. Preferably, the concentration of TFA in this step is from about 0.01M to about 0.2M. The use of TFA or an equivalent acid at this stage significantly facilitates cleavage.

In its more broad aspects, the invention includes cleavage reagents having the formula R₃SiO⁻X⁺, in which R is a straight or branched chain hydrocarbon radical having from 1 to 10 carbon atoms and X is an alkali metal ion, preferably a sodium or a potassium ion.

The broader aspects of the invention also include trimethylamine N-oxides in which the alkyl groups have from one to four carbon atoms. Triethyl, tripropyl, triisopropyl, tributyl or triisobutyl amine N-oxides may be used. Such reagents have the formula (R₁)₃ N⁺-O⁻, in which R₁ is an alkyl group having from one to four carbon atoms.

Suitable supports include activated carboxylic acid modified non-porous preferably polyethylene films or membrane. This support for C-terminal sequencing, hereinafter generally identified as PE-COOH, offers a number of advantages over the existing supports. These include: (1) stability of the support to the conditions employed for C-terminal sequencing; (2) the ability, due to the hydrophilic nature of the surface groups, to use both aqueous and organic solvents for performing chemistry on covalently coupled polypeptide samples; (3) a high capacity (3.2 nmoles/mm² of surface area) to covalently couple polypeptides; (4) the convenient size (1 x 5 mm) of the support needed for sequencing is similar to that employed in applicant's continuous flow reactor for automated N-terminal sequencing.

The PE-COOH films useful as supports in the present invention are typically 25.4 µm (1 mil) thick with a range of from 12.7 to 508 µm (0.5 to 20 mils) thick. The films are non-porous and naturally hydrophobic. A preferred method for preparing PE-COOH from polyethylene films is described in detail in U.S. Patent Number 4,339,473. These PE-COOH films are commercially available from Pall Corporation, 30 Sea Cliff Avenue, Glen Cove, New York 11542 under the tradename PERMION.

PE-COOH such as the "PERMION" (trademark) product of Pall Corporation is activated by the formation of an ester derivative with which the H-terminus of the peptide sample may be coupled. Appropriate activation reagents include 1,3-dicyclohexylcarbodiimide (DCC), 1,1' carbonyldiimidazole (CDI), l-ethyl-3-(3-dimethylamiopropyl) carbodiimide hydrochloride (EDC), benzotriazol-1-yl-oxy-tris(dimethylamino)phosphonium hexafluorophosphate (BOP), 1,3-Diisopropylcarbodiimide (DICD).

In general, activation of PE-COOH is accomplished by reaction for an appropriate time, e.g. about two hours, with a solution of an activating agent in a suitable solvent at about room temperature.

Table I is illustrative.

**TABLE I**

| Effect of Different Coupling Reagents And Solvents on the Yield of Leucine Enkephalin Coupled to PE-COOH | | |
|---|---|---|
| Activating Reagent | nmol covalently coupled to membrane (% yield) | |
| | 50% acetonitrile | 50% DMF |
| DCC | 0.2 (4.4) | 1.8 (39.6) |
| CDI | 0.07 (1.5) | 0.23 (5.1) |
| EDC | 0.32 (6.9) | 0.12 (2.6) |
| BOP | 0.58 (12.7) | 0.29 (6.4) |
| DICD | 0.68 (15.0) | 0.57 (12.6) |
| DCC - 1,3-dicyclohexylcarbodiimide CDI - 1,1' carbonyldiimidazole EDC - 1-ethyl-3-(3-dimethylamiopropyl) carbodiimide hydrochloride BOP - benzotriazol-1-yl-oxy-tris(dimethylamino) phosphonium hexafluorophosphate DICD - 1,3-Diisopropylcarbodiimide | | |

A strip of PE-COOH (1 cm x 0.1 cm) was placed in a 1.5 cm continuous flow reactor (CFR) (Shively, et al., 1987) sealed at one end. The membranes were reacted with 30 µl (30 mmol) of the activating reagent for 30 min. at room temperature. The solvent used in all cases was 100% DMF, except for when EDC was the activating agent, when water was the solvent. After the activation reaction, excess reagent was washed away with the solvent used for the activation reaction (3 ml). Each sample was then washed with acetonitrile (1 ml) and dried in a vacuum centrifuge. The activated membranes were then reacted with a 30 µl solution (4.54 nmol) of the peptide, YGGFL, in either 50% aqueous DMF or 50% aqueous acetonitrile in a CFR reactor (sealed at one end) for 16 hours. The membranes were washed with either 50% acetonitrile or 50% DMF, followed by acetonitrile, and then dried in a vacuum centrifuge. The amount of coupled peptide was determined by amino acid compositional analysis of the derivatized membrane.

Table II reports the effect of activation time and coupling time on the yield of covalent attachment of YGGFL.

**TABLE II**

| Effect of Activation Time and Coupling Time on the Yield of Covalent Attachment of YGGFL | | | |
|---|---|---|---|
| Activation Time (hrs) | Coupling Time (hrs) | Amount Coupled (nmol) | Yield |
| 0.5 | 1 | 0.54 | 4.0 |
| 0.5 | 2 | 2.11 | 15.6 |
| 0.5 | 4 | 3.33 | 24.7 |
| 0.5 | 20 | 5.19 | 38.4 |
| | | | |
| 1.0 | 1 | 1.01 | 7.5 |
| 1.0 | 2 | 1.59 | 11.8 |
| 1.0 | 4 | 2.73 | 20.2 |
| 1.0 | 20 | 5.57 | 41.3 |
| | | | |
| 2.0 | 1 | 2.44 | 18.1 |
| 2.0 | 2 | 2.45 | 18.1 |
| 2.0 | 4 | 5.25 | 38.9 |
| 2.0 | 20 | 7.2 | 53.3 |
| | | | |
| 4.0 | 1 | 0.52 | 3.9 |
| 4.0 | 2 | 0.52 | 3.9 |
| 4.0 | 4 | 3.4 | 25.2 |
| 4.0 | 20 | 4.1 | 30.4 |

The membranes (1 x 12.5 mm) were activated by an excess of DCC in anhydrous DMF (1 g/l ml) for 2 hours. At the end of the activation reaction, the excess reagent was removed with anhydrous DMF and the activated membrane strip dried in a vacuum centrifuge. Each activated membrane was inserted into a continuous flow reactor (CFR) (Shively et al., Analytical Biochemistry (1987) 163, 517-529) containing a 100 µl solution of leucine enkephalin (13.5 nmol) in 50% aqueous DMF for the indicated time at 22°C. The microbore tubing on one end of the CFR was sealed by first heating and then pinching closed with pliers. After the coupling reaction, the membrane was rinsed with coupling solvent and acetonitrile, and then dried in a vacuum centrifuge. The amount of covalently coupled peptide was determined by amino acid analysis.

An important aspect of the invention, preferred for the C-terminal sequencing of peptides consisting of a large number of amino acid residues entail the addition of a linker arm, of the general formula NH₂(CH₂)n COOH, wherein n is between 4 and 10 to the PE-COOH carboxyls followed by covalent attachment of the peptide sample to the linker arm carboxyl. Where linker arms are utilized the PE-COOH carboxyls are preferably activated with 2-fluoro-1-methylpyridine to facilitate attachment to the linker molecule. See generally Mukaiyama, Angewandte Chemie 18:707-721 (1979). The linker carboxyls are activated in the manner described for PE-COOH carboxyls.

### EXPERIMENTAL PROCEDURES

Materials*.* Acetic anhydride was purchased from Fisher Chemical Co. Trimethylsilylisothiocyanate (TMS-ITC), anhydrous dimethylformamide (DMF), Benzotriazol-1-yl-oxy-tris(dimethylamino)phosphoniumhexaflurophosphate (BOP), 1,3-Diisopropylcarbodiimide (DICD), and 1,1'-Carbonyldiimidazole (CDI) were from Aldrich. Water was purified on a Millipore Milli Q (tradename) system. All of the peptides used in this study were either obtained from Bachem or Sigma. 1,3-Dicylcohexylcarbodiimide (DCC), 1-ethyl-3-(3-dimethylaminopropyl)-carbodiimide (EDC), and triethylamine (sequanal grade) were obtained from Pierce. The carboxylic acid modified polyethylene membranes (PE-COOH) were provided by the Pall Corporation (Long Island, NY). The amino acid thiohydantoins used in this study were synthesized as described (Bailey and Shively, Biochemistry 29:3145-3156 (1990).

Covalent Coupling of Peptides to Carboxylic Acid Modified PVDF. A piece of PVDF-COOH (1 cm x 0.1 cm) was placed in a continuous flow reactor (CFR) (Shively, et al. Anal. Biochem. 163:517-529 (1987)), 2 µl of 1,3-dicyclohexylcarbodiimide (DCC) (2 mmol/ml) in N,N-dimethylformamide (DMF) was added through a Hamilton (tradename) syringe. The microbore tubing on one end of the CFR was sealed by first heating and then pinching closed with pliers. The CFR was then placed in a sealed eppendorf tube. After a two hour activation (25°C), the activated membrane was rinsed with 2 ml of DMF and then dried under a stream of argon. Leucine enkephalin (2 µl of a 14 nmol/µL solution in DMF) was carefully layered on the dry membrane and allowed to react overnight. The membrane was washed with methanol (5 ml) and dried under a stream of argon. The yield of coupled peptide was determined, after acid hydrolysis of the derivatized membrane, by amino acid composition analysis. Typical yields were approximately 50%.

Covalent Coupling of Peptides to Carboxylic Acid Modified Polyethylene. A piece of PE-COOH (1 x 12.5 mm) was placed in a continuous flow reactor (CFR), (Shively, et al., (1987) supra), and activated with an excess of 1,3-dicylohexylcarbodiimide in anhydrous DMF (1 g/ml) for 2 hours. The microbore tubing on one end of the CFR was sealed by first heating and then pinching closed with pliers. The CFR was then placed in a sealed eppendorf tube. At the end of the activation reaction, the excess reagent was washed off with anhydrous DMF and dried in a vacuum centrifuge. Each activated membrane was again inserted into a continuous flow reactor (CFR) (Shively, et al., containing the peptide solution of interest and incubated overnight (20 hours) at 22°C. The capacity of the CFR used was 100 µl. After the coupling reaction, the membrane was rinsed with coupling solvent and acetonitrile, and then dried in a vacuum centrifuge.

Use of this new reagent for cleavage in a 1:1 solution of methanol and tert-butanol as a solvent has allowed the automated sequencing of several peptides covalently coupled to a support such as a polyethylene or PVDF membrane. Examples are shown for the peptides, KVILF, YGGFMRGL, YGGFL, and RGYALG. In this case the thiohydantoin of arginine co-elutes with the only major background peak. The combination of methanol with tert-butanol is the preferred solvent for the cleavage reaction. Other alcoholic solvents useful in the invention include alkanols having from one to six atoms, e.g. ethanol, isopropanol, 2-trimethylsilylethanol and isobutanol.

### EXEMPLIFICATION OF THE C-TERMINAL SEQUENCING INVENTION

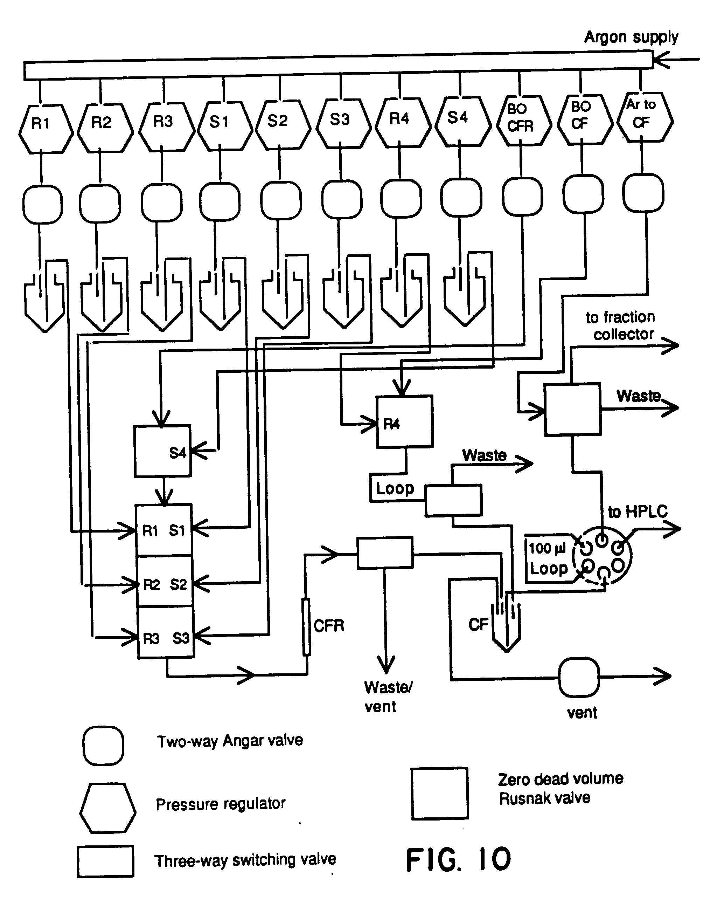

The examples described herein illustrate C-terminal sequencing accomplished with a modified N-terminal sequencer based on the design of Hawke, et al., Anal.Biochem. 147:315-330 (1985), in which a continuous flow reactor as described in Shively, EP-A-299741, was utilized. Figure 10 is the schematic of the instrument used to perform all of the examples. Release of thiohydantoin amino acids was detected by an on-line HPLC. See Shively, Anal.Biochem. 163:517-529 (1987). The reagent and solvent delivery system were all constructed from perfluoro-elastomers. Delivery valves as generally described in United States patent 4,530,586 provided with electromagnetically actuated solenoids and zero dead volume were connected in a modular fashion providing multiple input to a single output line. The valves which control the flow of reagents and solvents are computer operated pursuant to a program appropriate for the chemistry involved.

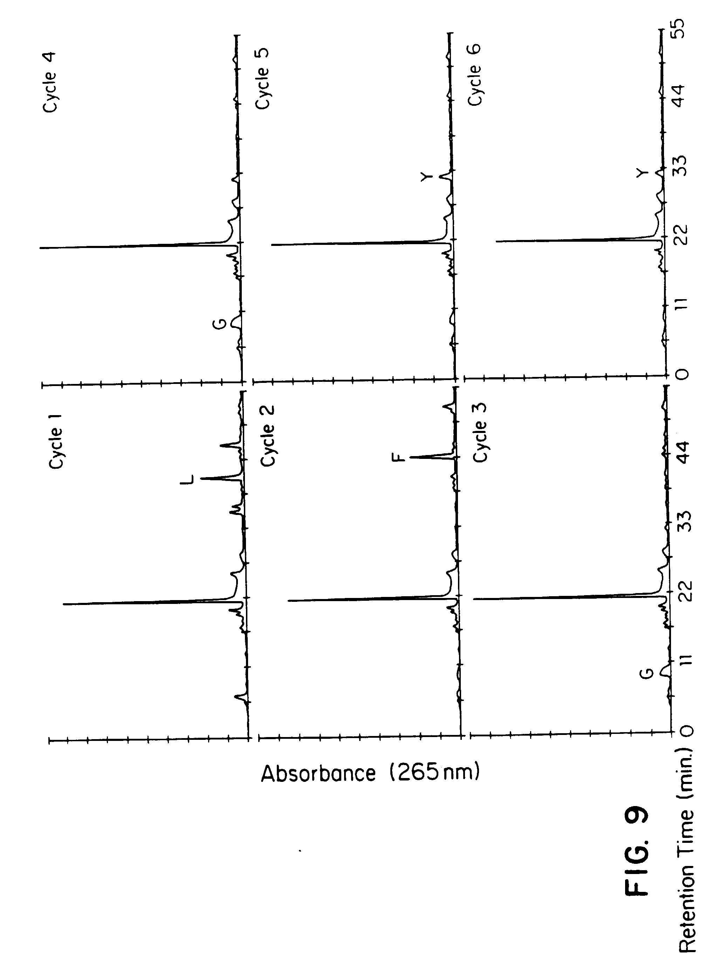

Table III is a program summary for the C-terminal sequencer illustrated by Figure 9 and used to perform the experiment described in the Example.

**Table III**

| C-Terminal Sequencer Program Summary | | |
|---|---|---|
| Continuous Flow Reactor (CFR) (65°C) | Conversion Flask (CF) (55°C) | Duration (sec) |
| S1 reaction | | 120 |
| S1 reaction | | 120 |
| | R4 rinse | 120 |
| Dry | Dry | 100 |
| R1 reaction | | 180 |
| R2 reaction | | 450 |
| S4 rinse | | 60 |
| R1 reaction | | 180 |
| R2 reaction | | 450 |
| S4 rinse | | 60 |
| R1 reaction | | 180 |
| R2 reaction | | 450 |
| S4 rinse | | 60 |
| R1 reaction | | 180 |
| R2 reaction | | 450 |
| S4 rinse | | 120 |
| S2 rinse | | 60 |
| S4 rinse | | 120 |
| S3 rinse | | 120 |
| S4 rinse | | 120 |
| R3 reaction | | 1200 |
| R3 to CF | | 45 |
| R3 reaction | Dry | 240 |
| R3 reaction | Dry | 240 |
| S2 delivery | | 12 |
| S2 reaction | | 120 |
| S2 to CF | | 45 |
| S2 delivery | | 11 |
| S2 reaction | | 120 |
| S2 to CF | | 45 |
| | inject | 7 |
| pause | pause | 60 |

### EXAMPLE 1

### Sequencing of Leucine Enkephalin (YGGFL) (1.2 nmoL) Covalently Coupled to PVDF-COOH

The composition of the reagents and solvents is set forth in Table IV.

**TABLE IV**

| Composition of Reagents and Solvents | |
|---|---|
| R1 | Acetic anhydride |
| R2 | 30% TMS-ITC in acetonitrile |
| R3 | 0.05 M Sodium trimethylsilanolate in 2-trimethylsilylethanol |
| R4 | Methanol |
| S1 | Acetonitrile |
| S2 | 0.8% Trifluoroacetic acid in water |
| S3 | Methanol |
| S4 | ------ |

Activated PVDF-COOH understood to be described in U.S. patent 5,611,861, was obtained from Pall Corporation. The thiohydantoin amino acid derivatives were separated by reverse phase HPLC. This separation was performed on Phenomenex Ultracarb (tradename) 5 ODS(30) column (2.0 mm x 25 mm) on a Beckman System Gold (tradename) with a Shimadzu (SPD-6A) detector (tradename). The column was eluted for 2 min with solvent A (0.1% trifluoroacetic acid in water) and then followed by a discontinuous gradient to solvent B (80% acetonitrile, 10% methanol in water) at a flow rate of 0.15 mL/min at 22°C. The gradient used was as follows: 0% B for 2 min, 0-6% B over 3 min, 6-35% B over 35 min, 35-50% B over 10 min, and 50-0% B over 10 min.

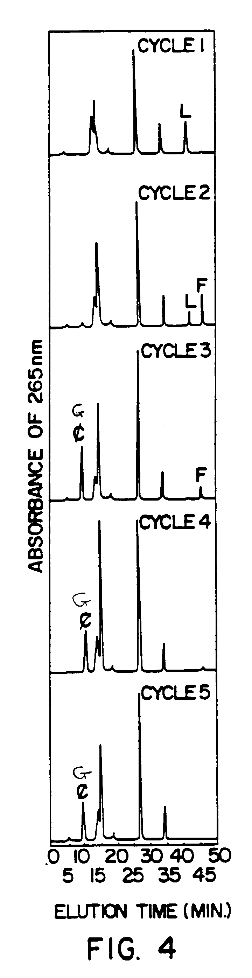

Figure 4 depicts the HPLC analysis of products of the reaction.

### EXAMPLE 2

### Sequencing of KVILF (1.2 nmoL) Covalently Coupled to PVDF-COOH

The composition of the reagents and solvents is set forth in Table V.

**TABLE V**

| Composition of Reagents and Solvents | |
|---|---|
| R1 | Acetic anhydride |
| R2 | 30% TMS-ITC in acetonitrile |
| R3 | 0.05 M Sodium trimethylsilanolate in methanol |
| R4 | Methanol |
| S1 | Acetonitrile |
| S2 | 0.8% Trifluoroacetic acid in water |
| S3 | ------ |
| S4 | ------ |

The thiohydantoin amino acid derivatives were separated by reverse phase HPLC. This separation was performed on Phenomenex Ultracarb 5 ODS(30) column (2.0 mm x 25 mm) on a Beckman System Gold with a Shimadzu (SPD-6A) detector. The column was eluted for 2 min with solvent A (0.1% trifluoroacetic acid in water) and then followed by a discontinuous gradient to solvent B (80% acetonitrile, 10% methanol in water) at a flow rate of 0.15 mL/min at 22°C. The gradient used was as follows: 0% B for 2 min, 0-6% B over 3 min, 6-35% B over 35 min, 35-50% B over 10 min, and 50-0% B over 10 min.

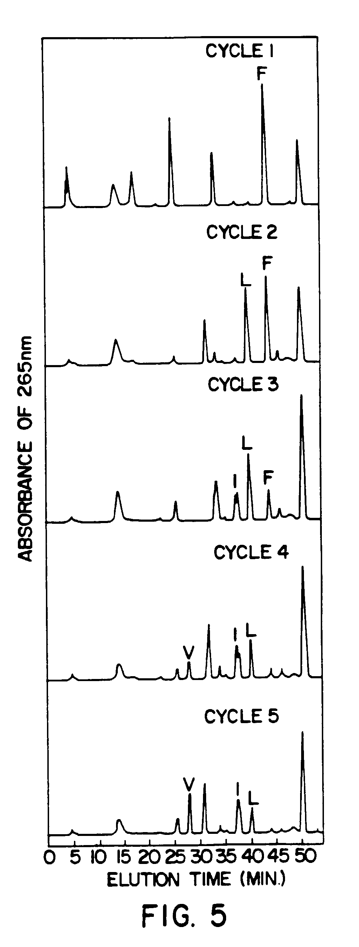

Figure 5 depicts the HPLC analysis of products of the reaction.

### EXAMPLE 3

### Sequencing of Leucine Enkephalin (YGGFL) (24.2 nmol) Covalently Coupled to Polyethylene

The composition of the reagents and solvents is set forth in Table VI.

**TABLE VI**

| Composition of Reagents and Solvents | |
|---|---|
| R1 | Acetic anhydride |
| R2 | 30% TMS-ITC in acetonitrile (v/v) |
| R3 | 0.10 M Sodium trimethylsilanolate in 50% methanol, 50% t-butyl alcohol |
| R4 | Methanol |
| S1 | 5% Triethylamine in water |
| S2 | 1.0% Trifluoroacetic acid in water |
| S3 | 50% Methanol, 50% Water |
| S4 | Acetonitrile |

The thiohydantoin amino acid derivatives were separated by reverse phase HPLC. The separation was performed on a Phenomenex Ultracarb 5 ODS(30) column (2.0 mm x 25 mm) on a Beckman 126 Pump Module with a Shimadzu (SPD-6A) detector. The column was eluted for 5 min with solvent A (0.006% phosphoric acid, 0.006% triethylamine, 0.045% pentanesulfonic acid) and then followed by a discontinuous gradient to solvent B (0.03% phosphoric acid, 0.045% triethylamine, 30% acetonitrile, 0.045% pentanesulfonic acid) at a flow rate of 0.15 mL/min at 22°C. The gradient used was as follows: 0% B for 5 min, 0-20% B over 7 min, 20-100% B over 25 min, 100% for 23 min, and 100-0% B over 2 min.

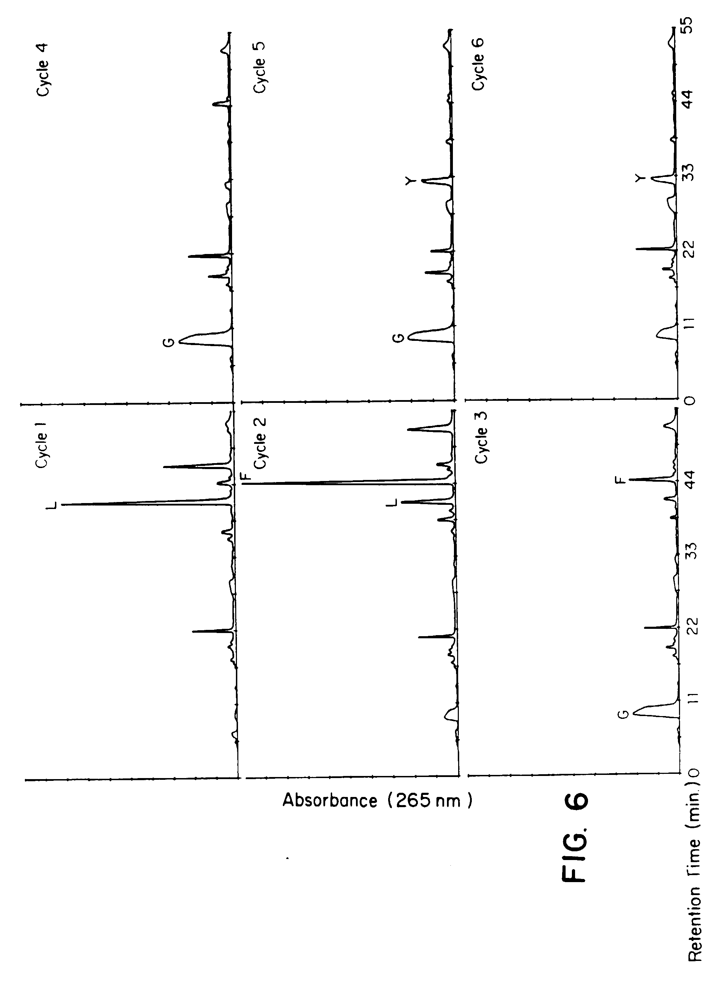

Figure 6 depicts the results of the HPLC analyses.

### EXAMPLE 4

### Sequencing of RGYALG (8.1 nmol) Covalently Coupled to PE-COOH

The sequencing of RGYALG was performed exactly as described in Example 3.

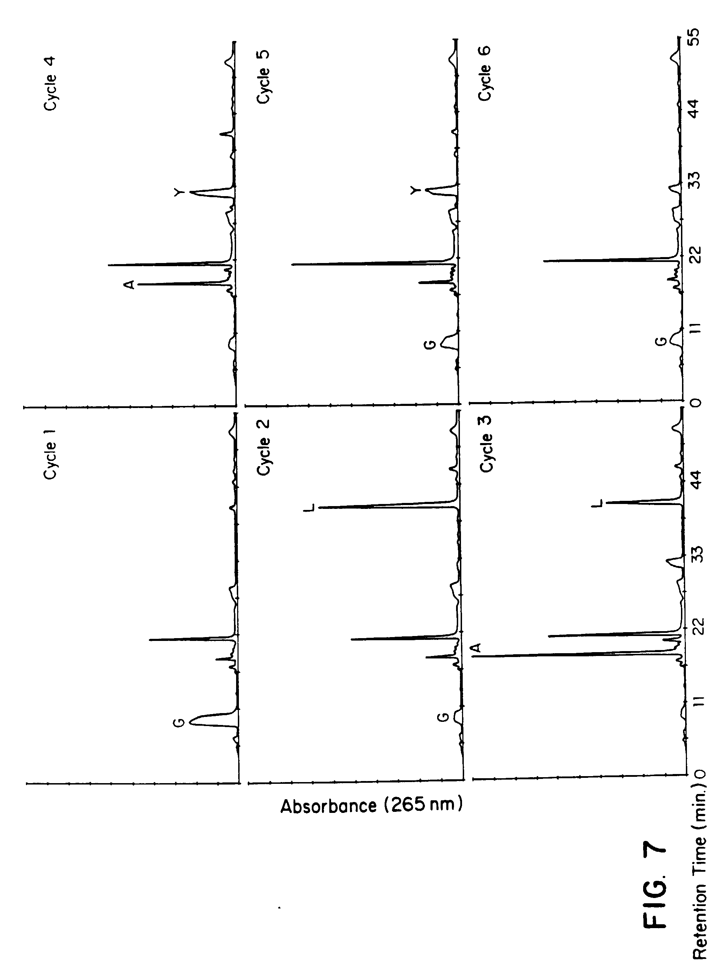

Figure 7 depicts the HPLC analyses.

### EXAMPLE 5

### Sequencing of YGGFMRGL (9.6 nmol) Covalently Coupled to PE-COOH

The sequencing of YGGFMRGL was performed exactly as described in Example 4.

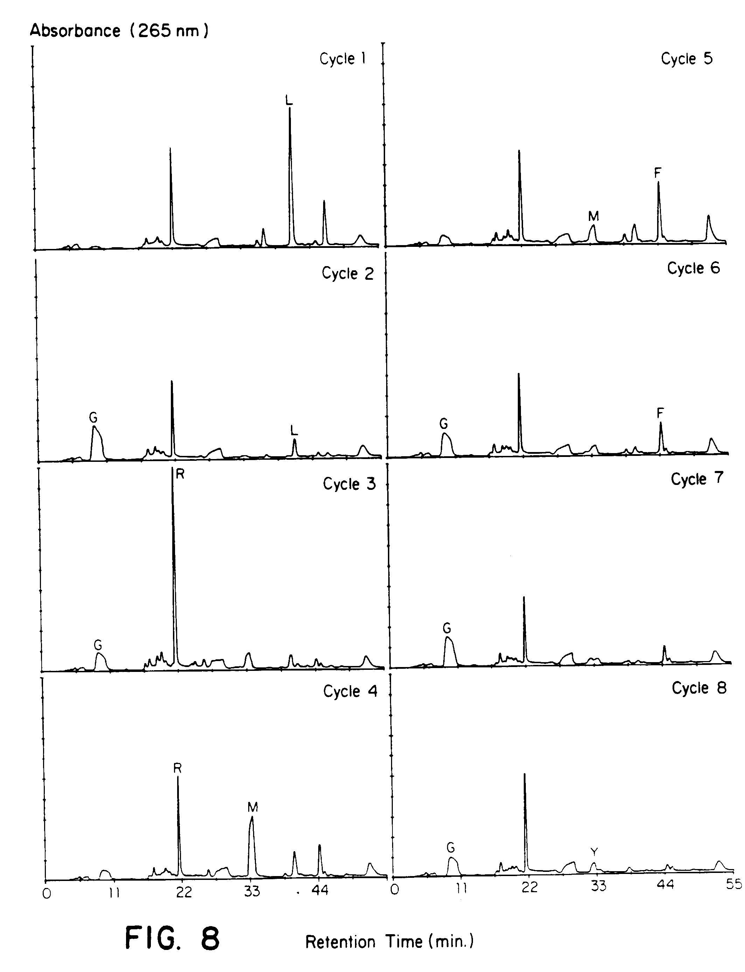

Figure 8 depicts the HPLC analyses.

### EXAMPLE 6

### Sequencing of YGGFL (3.7 mol) Covalently Coupled to the Linker 11-Aminoundecanoic Acid H₂N(CH₂)₁₀ CO₂H

Figure 9 depicts the sequencing from the C-terminal of the peptide YGGFL (3.7 mol) covalently coupled to the linker ll-aminoundecaonic acid H₂N (CH₂)₁₀ CO₂H. The linker was attached to PE-COOH by activation of the PE-COOH carboxyl groups with DCC as described. After attachment, the linker carboxyl groups were similarly activated with DCC and the peptide then covalently attached. In the absence of linkers after six cycles of sequencing, 50% of the N-terminal amino acid, Y, is still covalently attached when sequencing the peptide YGGFL (Figure 6). When the same peptide is sequenced after attachment to the above mentioned linker (Figure 10), using the same sequencing protocol, only 12% of the N-terminal amino acid, Y is still covalently attached.

The sequencing of YGGFL was performed exactly as described in Example 3.

## Claims

1. A method for sequencing a peptide by carboxyl terminal degradation comprising coupling the carboxyl terminus of a peptide with a silylisothiocyanate coupling reagent to form a peptidyl thiohydantoin derivative and cleaving the peptidyl thiohydantoin derivative with a reagent having the formula (R¹)₃N⁺O⁻ in which R¹ is an alkyl group having from 2 to 4 carbon atoms; to provide a thiohydantoin derivative of the amino acid previously at the carboxyl terminus of the peptide and a peptidyl residue lacking such an amino acid.

2. A method as claimed in claim 1, wherein the cleaving reagent is trimethylamine N-oxide.

3. A method as claimed in claim 1 or claim 2, wherein the peptide is coupled to a polyvinyldifluoride or a polyethylene membrane.

4. A method for sequencing a peptide by carboxyl terminal degradation comprising coupling the carboxyl terminus of a peptide with a silylisothiocyanate coupling reagent to form a peptidyl thiohydantoin derivative with a reagent having the formula R₃SiO⁻X⁺, in which R is a straight or branched chain hydrocarbon radical having from 1 to 10 carbon atoms and X is an alkali metal ion; or the formula (R¹)₃N⁺O⁻ as defined in claim 1; to provide a thiohydantoin derivative of the amino acid previously at the carboxyl terminus of the peptide and a peptidyl residue lacking such an amino acid,
wherein the peptide is coupled to a polyvinyldifluoride or a polyethylene membrane which is carboxylic acid modified and, optionally, activated.

5. A method as claimed in claim 4, wherein the cleaving reagent is sodium trimethylsilanolate or trimethylamine N-oxide.

6. A method as claimed in claim 4 or claim 5, in which the N-terminal of the peptide is coupled to an activated ester of the carboxylic acid modified polyethylene membrane and in which the activated ester is produced by reacting the carboxylic acid modified polyethylene membrane with 1,3-dicyclohaexylcarbodiimide (DCC), 1,1' carbonyldiimidazole (CDI), 1-ethyl-3-(3-dimethylamiopropyl) carbodiimide hydrochloride (EDC), benzotriazol-1-yl-oxytri (dimethylamino) phosphonium hexafluorophosphate (BOP), 1,3,-Diisopropylcarbodiimide (DICD) or 2-fluoro-1-methyl pyridine.

7. A method as claimed in claim 4 or claim 5, wherein said peptide is covalently attached to the carboxyl group of a linker which is coupled by an amide linkage to the surface carboxyl radicals of the activated carboxylic acid modified polyethylene membrane, wherein the linker, prior to the coupling, has the formula H₂NC(CH₂)ₙCOOH, in which n has a value from 4 to 10; the carboxyl of the linker being esterified by reaction with 2-fluoro-1-methyl pyridine.

8. A method as claimed in any of claims 1 to 7, wherein the cleaving of the peptidyl thiohydantoin is conducted in a solvent which is an alkanol having from 1 to 4 carbon atoms or trimethylsilylethanol.

9. A method as claimed in any of claims 1 to 8, wherein the silylisothiocyanate is trimethylsilyl-isothiocynate.

## Patentansprüche

1. Verfahren zum Sequenzieren eines Peptids durch den carboxylterminalen Abbau, enthaltend die Kopplung des Caboxylterminus eines Peptids mit einem Silylisothiocyanat-Kopplungsreagenz, um ein Peptidthiohydantoinderivat zu bilden und Spaltung des Peptidthiohydantoinderivats mit einem Reagenz der Formel (R¹ )₃N⁺O⁻, in dem R¹ eine Alkylgruppe mit 2 bis 4 Kohlenstoffatomen ist, um ein Thiohydantoinderivat der vorher am Carboxylterminus des Peptids gelegenen Aminosäure und einen eine solche Aminosäure fehlenden Peptidrest bereitszustellen.

2. Verfahren nach Anspruch 1, worin das Spaltreagenz Trimethylamin-N-oxid ist.

3. Verfahren nach Anspruch 1 oder 2, worin das Peptid an ein Polyvinyldifluorid- oder eine Polyethylenmembran gekoppelt ist.

4. Verfahren zum Sequenzieren eines Peptids durch carboxylterminalen Abbau, enthaltend die Kopplung des Caboxylterminus eines Peptids mit einem Silylisothiocyanat-Kopplungsreagenz, um ein Peptidthiohydantoinderivat mit einem Reagenz der Formel R₃SiO⁻X⁺ zu bilden, in welchem R ein gerad- oder verzweigkettiger Kohlenwasserstoffrest mit 1 bis 10 Kohlenstoffatomen und X ein Alkalimetallion ist, oder der Formel (R¹)₃N⁺O⁻ wie in Anspruch 1 definiert, um ein Thiohydantoinderivat der vorher an dem Carboxylterminus des Peptids gelegenen Aminosäure und einen eine solche Aminosäure fehlenden Peptidrests bereitzustellen, worin das Peptid an ein Polyvinyldifluorid- oder eine Polyethylenmembran, welche carbonsäuremodifiziert und gegebenenfalls aktiviert ist, gekoppelt ist.

5. Verfahren nach Anspruch 4, worin das Spaltreagenz Natriumtrimethylsilanolat oder Trimethylamin-N-oxid ist.

6. Verfahren nach Anspruch 4 oder 5, in welchem der N-Terminus des Peptids an einen aktivierten Ester der carbonsäuremodifizierten Polyethylenmembran gekoppelt ist und in welchem der aktivierte Ester durch Umsetzung der cabonsäuremodifizierten Polyethylenmembran mit 1,3-Dicyclohexylcarbodiimid (DCC), 1,1-Carbonyldiimidazol (CDI), 1-Ethyl-3-(3-dimethylaminopropyl)carbodiimiidhydrochlorid (EDC), Benzotriazol-1-yl-oxy-tri(dimethylamino) phosphoniumhexafluorphosphat (BOP), 1,3-Diisopropylcarbodiimid (DICD) oder 2-Fluor-1- methylpyridin hergestellt ist.

7. Verfahren nach Anspruch 4 oder 5, worin das Peptid kovalent an die Carboxylgruppe eines Binders angehaftet ist, welcher über eine Amidbindung an die Oberflächencarboxylreste der aktivierten carbonsäuremodifizierten Polyethylenmembran gekoppelt ist, worin der Binder vor der Kopplung die Formel H₂NC(CH₂)ₙCOOH auweist, in welchem n einen Wert von 4 bis 10 hat, wobei das Carboxyl des Binders durch Umsetzung mit 2-Fluor-1-methylpyridin verestert worden ist.

8. Verfahren nach einem der Ansprüche 1 bis 7, worin die Spaltung des Peptidthiohydantoins in einem Lösungsmittel durchgeführt wird, welches ein Alkanol mit von 1 bis 4 Kohlenstoffatomen oder Trimethylsilylethanol ist.

9. Verfahren nach einem der Ansprüche 1 bis 8, worin das Silylisothiocyanat Trimethylsilylisocyanat ist.

## Revendications

1. Procédé pour séquencer un peptide par dégradation du site carboxyle terminal, qui comprend l'étape consistant à coupler le site carboxyle terminal d'un peptide avec un réactif de couplage de type silylisothiocyanate, pour former un dérivé de peptidylthiohydantoïne, et à cliver le dérivé de peptidylthiohydantoïne avec un réactif de formule (R¹)₃N⁺O⁻ dans laquelle R¹ est un groupe alkyle ayant de 2 à 4 atomes de carbone ; pour donner un dérivé thiohydantoïnique de l'acide aminé précédemment au niveau du site carboxyle terminal du peptide et un résidu peptidyle ne comportant pas un tel acide aminé.

2. Procédé selon la revendication 1, dans lequel le réactif de clivage est le N-oxyde de triméthylamine.

3. Procédé selon la revendication 1 ou 2, dans lequel le peptide est couplé à un poly(difluorure de vinyle) ou à une membrane de polyéthylène.

4. Procédé pour séquencer un peptide par dégradation du site carboxyle terminal, qui comprend l'étape consistant à coupler le site carboxyle terminal d'un peptide avec un réactif de couplage de type silylisothiocyanate, pour former un dérivé de peptidylthiohydantoïne avec un réactif de formule R₃SiO⁻X⁺, dans laquelle R est un radical hydrocarboné à chaîne droite ou ramifiée ayant de 1 à 10 atomes de carbone, et X est un ion d'un métal alcalin ; ou encore de formule (R¹)₃N⁺O⁻ selon la revendication 1 ; pour donner un dérivé thiohydantoïnique de l'acide aminé précédemment au niveau du site carboxyle terminal du peptide et un résidu peptidyle ne comportant pas un tel acide aminé,
dans lequel le peptide est couplé à un poly(difluorure de vinyle) ou à une membrane de polyéthylène qui a subi une modification par un acide carboxylique, et éventuellement une activation.

5. Procédé selon la revendication 4, dans lequel le réactif de clivage est le triméthylsilanolate de sodium ou le N-oxyde de triméthylamine.

6. Procédé selon la revendication 4 ou 5, dans lequel le site N-terminal du peptide est couplé à un ester activé d'une membrane de polyéthylène modifiée par un acide carboxylique, et dans lequel l'ester activé est produit par la réaction de la membrane de polyéthylène modifiée par un acide carboxylique avec du 1,3-dicyclohexylcarbodiimide (DCC), du 1,1'-carbonyldiimidazole (CDI), du chlorhydrate de 1-éthyl-3-(3-diméthylaminopropyl)carbodiimide (EDC), de l'hexafluorophosphate de benzotriazole-1-yl-oxytri(diméthylamino)phosphonium (BOP), du 1,3-diisopropylcarbodiimide (DICD) ou de la 2-fluoro-1-méthylpyridine.

7. Procédé selon la revendication 4 ou 5, dans lequel ledit peptide est fixé par liaison covalente au groupe carboxyle d'un liant qui est couplé par une liaison amide aux radicaux carboxyle en surface de la membrane de polyéthylène activée modifiée par un acide carboxylique, le liant, avant le couplage, ayant la formule H₂NC(CH₂)ₙCOOH, dans laquelle n a une valeur de 4 à 10 ; le groupe carboxyle du liant étant estérifié par une réaction avec la 2-fluoro-1-méthylpyridine.

8. Procédé selon l'une quelconque des revendications 1 à 7, dans lequel le clivage de la peptidylthiohydantoïne est mis en oeuvre dans un solvant qui est un alcanol ayant de 1 à 4 atomes de carbone ou le triméthylsilyléthanol.

9. Procédé selon l'une quelconque des revendications 1 à 8, dans lequel le silylisothiocyanate est l'isothiocyanate de triméthylsilyle.
